# FASCICULE DE BREVET EUROPEEN

(11) **EP 2 572 751 B1**
(45) Date de publication et mention de la délivrance du brevet: **29.10.2014**
(21) Numéro de dépôt: 12183715.7
(22) Date de dépôt: 10.09.2012
(51) Int. Cl.: A61N 1/05

(54) **Sonde de stimulation en zone étendue d'une cavité du coeur, implantable par filoguidage dans le réseau coronarien profond**
Sonde zur Stimulation einer ausgedehnten Zone in einem Hohlraum des Herzens, die per OTW-Katheterführung in das Netz der tiefen Herzkranzgefäße implantiert werden kann
Probe for stimulation in an extended region of a cardiac chamber, which can be implanted by wire guidance in the deep coronary network

(30) Priorité: 21.09.2011 FR 1158417
(43) Date de publication de la demande: 27.03.2013
(73) Titulaire: Sorin CRM SAS, 92140 Clamart (FR)
(72) Inventeur: Ollivier, Jean-François, 91190 Villiers Le Bacle (FR)
(74) Mandataire: Dupuis-Latour, Dominique

(56) Documents cités:
- EP-A1- 1 374 945
- US-A- 5 755 765
- US-A1- 2008 039 916
- US-A1- 2011 072 659

## Description

L'invention concerne les "dispositifs médicaux implantables actifs" tels que définis par la Directive 90/385/CEE du 20 juin 1990 du Conseil des communautés européennes, notamment les implants permettant de surveiller en continu le rythme cardiaque et délivrer si nécessaire au coeur des impulsions électriques de stimulation ou de resynchronisation.

Elle concerne plus précisément les sondes de stimulation cardiaque destinées à être implantées dans le réseau coronarien du coeur pour permettre la stimulation d'une cavité gauche ou droite, ventricule ou oreillette.

A la différence des cavités droites pour lesquelles il suffit d'implanter des sondes endocavitaires via le réseau veineux périphérique droit, la mise en place de sondes permanentes dans une cavité du coeur gauche impliquerait des risques opératoires importants, par exemple le risque de passage de bulles vers le réseau vasculaire cérébral situé en aval du ventricule gauche.

Pour cette raison, lorsque l'on veut stimuler une cavité gauche, on choisit le plus souvent d'introduire une sonde non pas dans la cavité à stimuler mais dans le réseau coronarien, la sonde étant pourvue d'une électrode qui sera appliquée contre la paroi de l'épicarde et orientée vers le ventricule gauche ou l'oreillette gauche, selon le cas. Ces sondes stimulent le muscle cardiaque via une ou plusieurs électrodes ponctuelles dont la position est fonction de la trajectoire prédéfinie de la veine canulée.

Une sonde de ce type est par exemple le modèle *Situs LV,* commercialisé par Sorin CRM (Clamart, France) et décrit dans le EP 0 993 840 A1 (ELA Medical).

Les US 2008/0039916 A1 et US 5 755 735 A divulguent d'autres exemples de telles sondes implantables dans le réseau coronaire. Le US 2011/0072659 A1, quant à lui, divulgue un autre type de sonde, pour la stimulation profonde du cerveau.

L'introduction d'une telle sonde se fait par le sinus coronaire, depuis son débouché dans l'oreillette droite. La sonde est ensuite poussée et orientée le long du réseau des veines coronaires jusqu'au site choisi. Cette intervention est très délicate compte tenu des particularités du réseau veineux et de ses voies d'accès, avec notamment le passage de valvules et tortuosités ainsi que la diminution progressive de diamètre du conduit au fur et à mesure de la progression de la sonde dans la veine coronaire sélectionnée.

Une fois la veine cible atteinte, le chirurgien recherche un site de stimulation satisfaisant, avec un bon contact électrique de l'électrode de stimulation contre le tissu de l'épicarde, ce contact devant être maintenu malgré les variations ou sollicitations diverses au cours du temps.

Il a été proposé de disposer plusieurs électrodes le long du corps de sonde pour augmenter les chances d'un compromis acceptable, en donnant éventuellement au corps de sonde une conformation particulière. Le chirurgien peut ainsi sélectionner, parmi les diverses électrodes présentes sur le corps de sonde, celle procurant la meilleure efficacité sur le plan électrique et hémodynamique. Une telle sonde à électrodes multiples est décrite notamment dans le EP 1 938 861 A1 (ELA Medical) et dans le US 2008/0039916 A1 précité.

Ces sondes permettent en particulier de mettre en oeuvre le concept de "repositionnement électronique", visant à diriger ou rediriger le champ électrique entre différentes électrodes disposées le long de la sonde de stimulation de la cavité gauche et/ou avec une des électrodes de la sonde de stimulation de la cavité droite. Cette technologie permet de gérer les micro-déplacements ou les évolutions du comportement hémodynamique (*reverse modeling),* simplement par reprogrammation du générateur par voie télémétrique à travers la peau, donc sans réintervention chirurgicale lourde.

La contrepartie de cette solution est une complexité grandissante de la structure de la sonde : la multiplication du nombre d'électrodes entraîne une multiplication du nombre de composants, et donc des liaisons électriques, ou nécessite le recours à des circuits de multiplexage pour la sélection des diverses électrodes présentes sur la sonde. Tout ceci conduit à un accroissement des risques mécaniques de rupture.

Le US 2009/157136 A1 décrit une technique de recherche d'un site de stimulation optimal au moyen d'un cathéter temporaire de *mapping* à introduire dans le sinus coronaire. Ce cathéter est soit un tube flexible ouvert à ses deux extrémités, soit un fil-guide. Dans l'un ou l'autre cas, il est doté en partie distale de multiples électrodes électriquement indépendantes, et en partie proximale d'un connecteur de liaison à un système d'acquisition qui permettra d'identifier le meilleur site de stimulation par un algorithme basé sur le mouvement cardiaque. Une sonde définitive multiélectrode permanente classique de diamètre standard 4,5 à 6 French (1,5 à 2 mm) est ensuite placée jusqu'à la position sélectionnée, via le guide (technique dite OTW, *Over The Wire* ou "filoguidage" classique), ou dans le tube du cathéter temporaire.

Une autre tendance des développements récents en matière de sondes de stimulation du ventricule gauche est la réduction du diamètre de la partie implantable dans le réseau coronarien, jusqu'à un diamètre de 4 French (1,33 mm). La taille du corps de sonde est en effet un facteur directement lié aux capacités de guidage contrôlé de la sonde dans le réseau veineux coronarien, de manière à pouvoir y sélectionner des sites de stimulation particuliers situés dans certaines veines collatérales. Ces sites sont atteints au moyen d'un cathéter de sous-sélection de veine servant à la mise en place d'un mandrin de guidage jusqu'au site choisi. Une fois la veine sélectionnée et le mandrin posé, le chirurgien fait progresser le corps de sonde qu'il glisse sur le mandrin, ce dernier jouant le rôle d'un fil support de faible diamètre guidant axialement le corps de sonde jusqu'à l'emplacement choisi (technique OTW de "filoguidage").

Le US 2008/0039916 A1 précité propose de réduire la partie d'extrémité distale à un diamètre de 1 à 5 French. Toutefois, la multiplication des électrodes et des composants ou conducteurs internes implique nécessairement un accroissement du diamètre du corps de sonde et diminue sa flexibilité, rendant difficile voire impossible le passage des tortuosités, ce qui va à l'encontre de la recherche d'un faible diamètre et d'une très grande souplesse distale, caractéristiques indispensables pour pouvoir atteindre les veines collatérales les plus profondes.

L'invention vise à s'affranchir de cette limitation, par une sonde de stimulation d'une cavité cardiaque, notamment du ventricule gauche, dont la partie active présente un très faible diamètre, permettant d'exploiter toute la longueur de la veine et d'utiliser de façon optimale toutes les veines présentes dans la zone basale.

Un autre but de l'invention est de proposer une telle sonde qui opère sur une zone de stimulation élargie, permettant ainsi (contrairement aux sondes traditionnelles) de stimuler simultanément plusieurs points de l'épicarde.

On a en effet constaté que la multiplication des points de stimulation sur le ventricule gauche est un facteur permettant d'améliorer de façon substantielle la qualité de la resynchronisation.

Un autre but encore de l'invention est de proposer une telle sonde qui offre la possibilité de stimuler éventuellement deux zones distantes via deux veines distinctes, tout en gardant la simplicité d'implantation d'une sonde unique.

Or, sur ce dernier point, les études en cours montrent qu'il est très difficile d'implanter concurremment deux sondes dans le réseau veineux coronarien. Une alternative connue consiste à doter un corps de sonde de plusieurs électrodes, jusqu'à quatre dans certains modèles. Il s'agit toutefois de sondes de diamètre relativement important, de l'ordre de 4 French (1,33 mm) au moins, du fait de la complexité des composants et liaisons nécessaires à la sélection des électrodes pour le repositionnement électronique. On retrouve donc la limitation que l'on a exposée plus haut sur la finesse de la sonde. De plus, la position relative des couples d'électrodes sélectionnés est très limitée, ces couples d'électrodes étant en outre nécessairement positionnés dans la même veine coronaire, généralement la veine postéro-latérale.

Ainsi, l'un des buts principaux de l'invention est de proposer une nouvelle configuration de sonde de stimulation de très faible diamètre, implantable dans le réseau veineux coronarien, autorisant pour améliorer l'efficacité de la stimulation : (i) une possibilité d'implantation dans des veines profondes, (ii) un élargissement de la zone stimulée et en outre (iii) la possibilité de stimuler éventuellement deux zones distantes via deux veines distinctes.

Un autre but de l'invention est de proposer une telle sonde qui soit de structure simple (donc peu coûteuse à fabriquer, et présentant une fiabilité maximale) et qui s'affranchisse des problèmes liés à la conception et à l'emploi des sondes à électrodes multiples, dont on a exposé plus haut la complexité structurelle et fonctionnelle.

Un autre but encore de l'invention est de proposer une telle sonde qui puisse être posée par des techniques conventionnelles, bien connues des praticiens, ne nécessitant donc aucun apprentissage ou habileté technique supplémentaire.

Pour atteindre les buts précités, l'invention repose sur l'idée consistant à prolonger un corps de sonde de type en lui-même conventionnel, de diamètre extérieur compris typiquement entre 3 et 5 French (1 et 2,66 mm), par une extension tubulaire creuse de très faible diamètre extérieur, typiquement 1 à 3 French (0,33 à 1 mm), soit environ la moitié du diamètre typique des sondes coronaires actuelles, dont le diamètre de la partie distale est généralement compris entre 4 et 6 French (1,33 à 2 mm). Le diamètre intérieur de l'extrémité tubulaire de l'extension est, quant à lui, compatible avec le passage d'un fil-guide de faible diamètre, typiquement un fil-guide de 0,014 pouce, soit 1,1 French (0,356 mm).

Le très faible diamètre extérieur de l'extension permettra de canuler des veines très étroites du réseau coronarien, non exploitées à ce jour du fait de la taille excessive des sondes coronaires permanentes conventionnelles.

Une autre caractéristique importante de l'invention réside dans le fait que, dans la veine, l'extension n'est pas soumise au risque d'abrasion avec un autre appareil (notamment une autre sonde), contrairement à la portion de la sonde demeurant dans l'oreillette droite ou le trajet intra-veineux. Par voie de conséquence, l'épaisseur du revêtement extérieur électriquement isolant de l'extension peut être considérablement réduite, contribuant ainsi à la réduction générale du diamètre hors-tout de cette extension.

Encore une autre caractéristique importante de l'extension réside dans la présence à sa surface extérieure d'un conducteur électrique périphérique, qui est isolé à l'exception d'une pluralité de zones ponctuellement dénudées, constituant des électrodes respectives reliées électriquement ensemble.

Les parties dénudées formant électrodes sont avantageusement regroupées en une ou plusieurs zones actives de stimulation. Ces électrodes sont destinées à venir en contact avec la paroi d'une veine-cible du réseau coronarien pour permettre l'application, simultanément en plusieurs endroits, d'impulsions de stimulation sur la paroi de l'épicarde.

On notera que c'est le conducteur lui-même qui est utilisé comme électrode (par les parties dénudées), à la différence des sondes conventionnelles qui utilisent généralement des électrodes rapportées, structurellement distinctes du conducteur qui les relie au générateur couplé à la sonde.

Enfin, la construction et les matériaux de l'extension sont choisis de manière à conférer à celle-ci les propriétés requises pour lui permettre de progresser dans le réseau coronarien veineux après avoir été enfilée sur un fil-guide (technique OTW de "filoguidage"), à savoir : raideur axiale suffisante pour faciliter la transmission des efforts de bout en bout (propriété dite de *pushability*), flexibilité distale et faible coefficient de frottement de la lumière interne pour permettre un bon glissement sur le fil-guide (*trac-kability).*

On verra par ailleurs que la configuration de la sonde selon l'invention permet, dans un mode de réalisation avantageux, d'introduire l'extension dans une première veine (veine "aller") puis par une anastomose vers une deuxième veine (veine "retour") en remontant dans celle-ci. On a en effet constaté la présence très fréquente (typiquement 60 à 80 % de la population de patients) d'anastomoses distales dans le réseau veineux coronarien, c'est-à-dire qu'à l'extrémité de certaines veines existe un passage vers une autre veine, avec de ce fait possibilité de communication entre deux veines distinctes au niveau de l'anastomose, via leurs extrémités distales respectives.

La répartition des électrodes (les zones ponctuellement dénudées) sur l'extension pourra être choisie de telle sorte que les électrodes soient groupées en deux ensembles séparés, formant deux parties actives distinctes, l'une destinée à définir des sites de stimulation dans la veine aller et l'autre destinée à définir des sites de stimulation dans la veine retour. Ces deux ensembles d'électrodes sont séparés par une région isolée correspondant à la partie la plus distale de la veine aller, à la région de l'anastomose et à la partie la plus distale de la veine retour.

La configuration des électrodes de l'extension permet ainsi, avec une unique sonde, de stimuler concomitamment deux zones relativement distantes, car situées dans deux veines distinctes. Le double effet d'éloignement de ces deux zones et de multiplication des points de stimulation dans chacune des zones procure un effet particulièrement bénéfique pour la resynchronisation du fonctionnement du coeur.

Enfin, comme on le verra plus bas, la sonde de l'invention (constituée par le corps de sonde et son extension portant les électrodes) pourra être posée par des techniques conventionnelles, bien connues des praticiens, ne nécessitant donc aucun apprentissage ou habileté technique supplémentaire.

La présente invention propose, plus précisément, une sonde de stimulation destinée à être implantée dans le réseau veineux coronarien pour la stimulation d'une cavité du coeur. Cette sonde comprend, de manière en elle-même connue et divulguée par exemple par le US 2008/0039916 A1 précité, un corps de sonde avec une gaine creuse traversée de part en part par une lumière centrale, l'extrémité distale du corps de sonde comprenant des moyens de retenue à une paroi du réseau coronarien. Le diamètre extérieur de l'extension est compris entre 1 et 3 French (0,33 et 1 mm). L'extrémité distale comporte à sa surface externe au moins un conducteur périphérique électriquement isolé à l'exception de zones ponctuellement dénudées destinées à venir en contact avec la paroi d'une veine cible du réseau coronarien, de manière à constituer ainsi un réseau d'électrodes de stimulation reliées électriquement ensemble. La sonde comporte une liaison électrique du conducteur périphérique à des moyens, disposés côté proximal du corps de sonde, de couplage à un générateur de dispositif médical implantable actif tel que stimulateur cardiaque ou resynchroniseur. Par ailleurs, le corps de sonde est prolongé à sa partie distale par une extension tubulaire creuse solidaire du corps de sonde et portant une partie active de la sonde, cette extension étant traversée de part en part par une lumière centrale communiquant avec la lumière centrale du corps de sonde de manière à y permettre l'introduction et le glissement d'un fil-guide pour l'implantation de la sonde par une technique de filoguidage.

De façon caractéristique de l'invention, l'extension porte à sa surface externe ledit conducteur périphérique électriquement isolé à l'exception desdites zones ponctuellement dénudées.

Le conducteur périphérique peut notamment être un fil bobiné sur un tube creux, ce fil étant électriquement isolé à l'exception des zones ponctuellement dénudées, formées côté extérieur du fil. Le fil est avantageusement un fil plat, pouvant notamment comprendre un coeur d'acier inoxydable gainé de platine-iridium et revêtu en surface d'une couche d'un matériau isolant. ce fil peut être bobiné en hélice à spires non jointives, l'intervalle entre spires étant garni d'un matériau isolant de remplissage sur l'épaisseur comprise entre la surface du tube creux et le niveau de la surface périphérique extérieure du fil bobiné.

Selon diverses autres caractéristiques subsidiaires avantageuses :
- la surface unitaire exposée des zones ponctuellement dénudées du conducteur périphérique est d'au plus 2 mm², et la surface totale exposée de ces zones est d'au plus 10 mm² ;
- les zones ponctuellement dénudées du conducteur périphérique sont regroupées sur au moins une région active s'étendant sur une longueur comprise entre 1 et 5 cm ;
- le corps de sonde porte côté distal au moins une électrode de stimulation bipolaire, non électriquement reliée au conducteur périphérique de l'extension ;
- les zones ponctuellement dénudées du conducteur périphérique sont regroupées sur deux régions actives s'étendant chacune sur une longueur comprise entre 1 et 5 cm et séparées par une région intermédiaire non active de longueur comprise entre 5 et 15 cm ;
- l'extension comporte à sa surface externe deux conducteurs périphériques électriquement distincts, chacun d'entre eux étant relié à des ensembles respectifs distincts de zones ponctuellement dénudées, une même région active comprenant au moins une zone ponctuellement dénudée de chaque ensemble, de manière à permettre une stimulation bipolaire entre ces dernières ;
- l'extension comporte à son extrémité distale au moins un marqueur radio-opaque de repérage de la position distale de l'extension ;
- les moyens de retenue à l'extrémité distale du corps de sonde comportent au moins un relief formé sur le corps de sonde, présentant localement un diamètre accru par rapport au diamètre propre du corps de sonde, notamment un relief hélicoïdal avec un filet s'enroulant autour du corps de sonde ;
- le diamètre du corps de sonde est compris entre 2 et 5 French (0,66 et 2,66 mm) ;
- le corps de sonde porte côté distal au moins un marqueur radio-opaque de repérage de la position proximale de l'extension.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon générale le myocarde, avec les principales veines du réseau coronarien dans lequel a été introduite une sonde selon l'invention, destinée à la stimulation du ventricule gauche.
La Figure 2 illustre une structure conventionnelle de sonde de stimulation du ventricule gauche selon l'état de la technique.
La Figure 3 est une vue d'un exemple de réalisation d'une sonde selon l'invention, montrant l'extrémité distale du corps de sonde prolongé par l'extension caractéristique de l'invention, l'ensemble étant enfilé sur un fil-guide en vue de son implantation.
Les Figures 4a à 4e illustrent les étapes successives de réalisation de l'extension d'une sonde selon l'invention.
La Figure 5 est une vue agrandie correspondant à la Figure 3, pour une variante portant sur la position des électrodes.
La Figure 6 est une vue agrandie et en coupe au niveau du détail repéré VI sur la Figure 5.
Les Figures 7a à 7d illustrent diverses configurations de sondes selon l'invention, pour une stimulation monopolaire (Figures 7a et 7c) ou bipolaire (Figure 7b et 7d), et pour des applications à une stimulation dans une seule veine (Figures 7a et 7b) ou simultanément dans deux veines différentes (Figure 7c et 7d).

On va maintenant décrire des exemples de réalisation de la sonde selon l'invention.

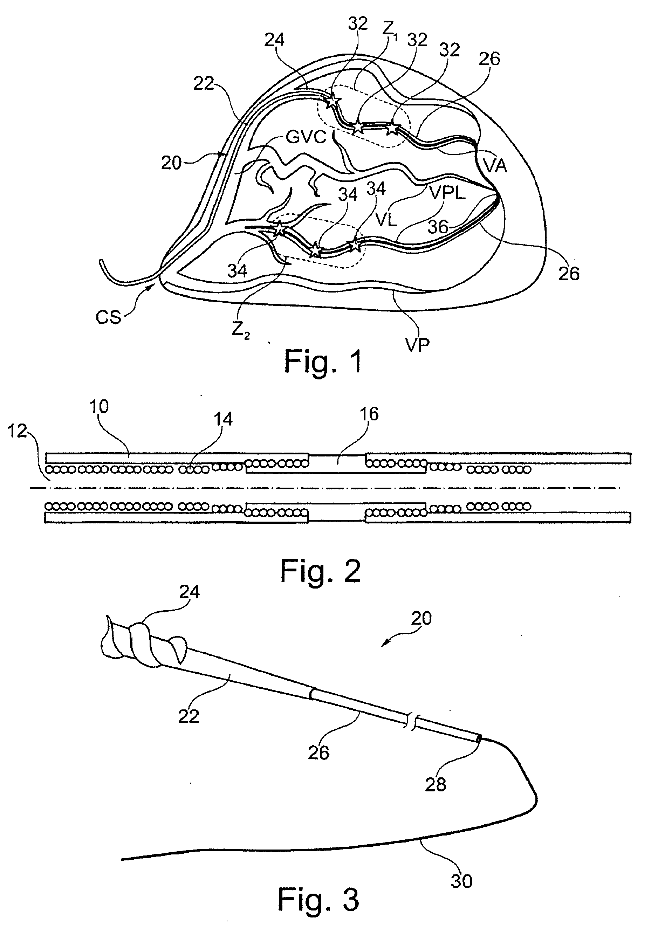

La Figure 1 illustre de façon générale le myocarde et les principaux vaisseaux du réseau coronarien, dans lequel on a introduit une sonde afin de stimuler le ventricule gauche.

Cette sonde est implantée par voie endocavitaire dans le réseau veineux coronarien via la veine cave supérieure, l'oreillette droite et l'entrée CS du sinus coronaire veineux. Le réseau veineux coronarien se développe ensuite en plusieurs branches à partir de la grande veine coronaire GVC, ces branches comprenant les veines postéro-latérale VPL, latérale VL, antéro-latérale VA et postérieure VP.

La Figure 2 illustre en coupe et de façon schématique, dans la région de son électrode de stimulation, la structure d'une sonde de stimulation conventionnelle destinée à être implantée dans le réseau veineux coronarien. Cette construction conventionnelle comprend un corps de sonde 10 électriquement isolant, avec une lumière centrale 12 permettant l'introduction d'un fil-guide servant à l'implantation de la sonde. La région interne de la sonde comprend également un conducteur spiralé 14 (typiquement en acier inoxydable MP35N) électriquement relié à une électrode 16 (typiquement en alliage de platine et d'iridium) rapportée sur le corps de sonde 10. Compte tenu des contraintes de dimensions du fil-guide, d'épaisseur de l'isolant, etc., le diamètre d'une telle sonde conventionnelle est généralement compris entre 4 et 6 French (1,33 et 2 mm), avec une lumière interne de 1,3 French (0,4 mm). De plus, la présence d'une électrode rapportée 16 introduit une discontinuité dans la structure de la sonde, ce qui peut avoir une conséquence sur l'endurance à long terme de cette dernière en cas de dimensionnement insuffisant des différents éléments.

Pour sa mise en place, cette sonde est introduite par le sinus coronaire CS et la grande veine coronaire GVC, puis dans la veine antérolatérale VA. Toutefois, compte tenu du diamètre externe hors-tout de la sonde, de 4,5 à 6 French (3 à 4 mm), il n'est pas possible d'atteindre les veines les plus profondes du réseau coronarien. La stimulation sera donc confinée aux veines principales, de plus grand diamètre, qu'il est possible d'atteindre.

La Figure 3 illustre la partie distale d'une sonde selon l'invention, enfilée sur un fil-guide en vue de son implantation. Cette sonde a été représentée également en configuration implantée, sur la Figure 1.

La sonde 20 selon l'invention comporte un corps de sonde 22 formé d'une gaine creuse tubulaire en matériau déformable, par exemple en silicone ou polyuréthanne, pourvue d'une lumière centrale s'étendant d'une extrémité à l'autre du corps de sonde 22.

Le diamètre extérieur hors-tout de ce corps de sonde 22 est compris entre 3 et 5 French (1 et 2,66 mm), de préférence non supérieur à 4 French (1,33 mm). Le corps de sonde 22 peut être éventuellement terminé dans sa région la plus distale par une partie de transition dont le diamètre se réduit progressivement, par exemple jusqu'à une valeur de 2 French (0,66 mm) pour permettre une pénétration plus profonde dans le réseau veineux.

Le diamètre externe de 4 French (33 mm) du corps de sonde procure une bonne résistance à l'abrasion et contribue à la stabilité mécanique de l'ensemble du système par effet de support mécanique dans le sinus coronaire.

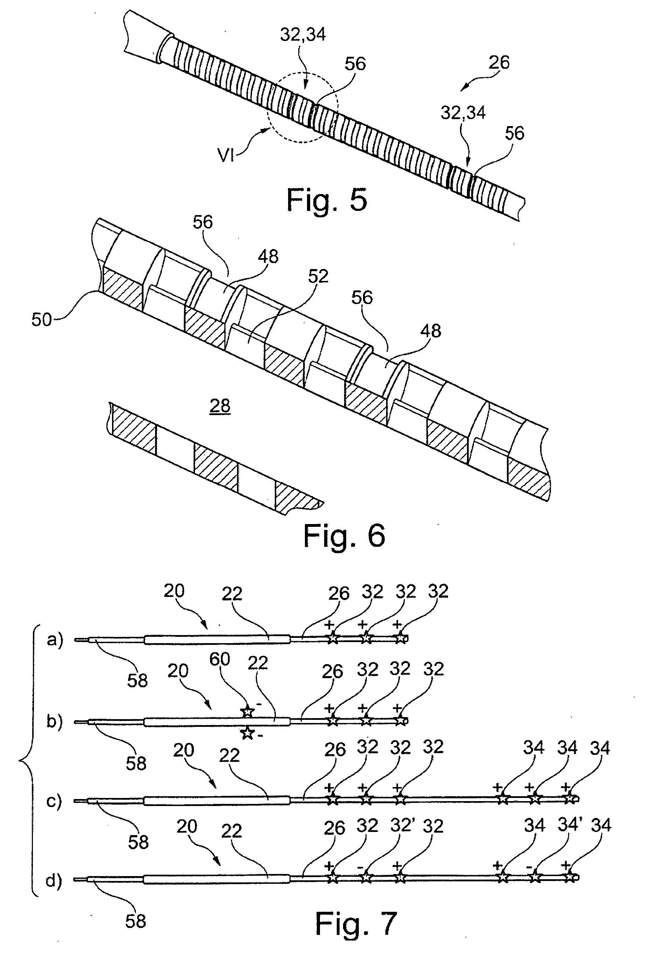

Le corps de sonde 22 loge un conducteur électrique interne (non illustré sur cette figure), qui débouche en partie proximale par un connecteur approprié (schématisé en 58 sur la Figure 7), par exemple de type IS-1, assemblé en usine.

À son extrémité opposée (distale), le corps de sonde 22 est pourvu d'un moyen de retenue 24 permettant son maintien mécanique dans la veine. Ce moyen de retenue est par exemple constitué d'une vis en silicone telle que celle décrite dans le EP 1 374 945 A1 (ELA Medical), avec un filet hélicoïdal s'enroulant autour du corps de sonde. Le filet est moulé monobloc avec le corps de sonde en élastomère de silicone, matériau peu traumatique et assurant une bonne biocompatibilité. Ce moyen de retenue 24 a été également schématisé sur la Figure 1. Il s'agit d'un moyen en lui-même connu, du même type par exemple que celui de la sonde *Situs LV* citée en introduction.

De façon caractéristique, le corps de sonde 22 est prolongé à sa partie distale par une extension tubulaire creuse 26 solidaire du corps de sonde et portant la partie active de la sonde. Cette extension tubulaire 26 est traversée de part en part par une lumière centrale communiquant avec la lumière centrale du corps de sonde, de manière à permettre l'introduction et le glissement d'un fil-guide 30 pour l'implantation de la sonde par une technique de filoguidage que l'on décrira plus bas. Le fil-guide 30 est pourvu à son extrémité distale d'une terminaison très souple pour ne pas être traumatique et permettre son introduction directe dans les vaisseaux du réseau coronarien sans risque de perforation. Une fois la sonde en place, ce fil-guide 30 qui ne sert qu'à l'implantation sera retiré par le chirurgien.

Le diamètre extérieur de l'extension tubulaire 26 est compris entre 1 et 3 French (0,33 et 1 mm), de préférence environ 2,1 French (0,7 mm), et le diamètre de la lumière centrale 28 doit permettre l'introduction d'un fil-guide 30 de diamètre typique (mais non limitatif) de 1 French (0,33 mm). Sur la Figure 1, cette sonde 20 a été illustrée telle qu'elle se présente *in situ,* après implantation. Le corps de sonde 22 pénètre dans le sinus coronaire CS et la grande veine cardiaque GVC jusqu'au débouché de la veine antérolatérale VA. L'extension tubulaire 26, quant à elle, est introduite profondément dans la veine antéro-latérale VA, et elle porte une pluralité d'électrodes de stimulation 32 (qui seront décrites en détail par la suite) destinées à stimuler le ventricule gauche à partir de plusieurs sites, symbolisés par des étoiles, localisés dans cette veine VA dans une première région active de stimulation Z₁.

Éventuellement, l'extension 26 porte également, à distance des électrodes 32, une autre série d'électrodes 34 destinées à stimuler le ventricule gauche depuis une autre veine, par exemple la veine postéro-latérale VPL via une communication par une anastomose 36 reliant la veine antérolatérale VA et la veine postéro-latérale VPL. L'extension tubulaire 26 traverse cette anastomose 36 et les régions les plus distales des deux veines VA et VPL le long d'une portion intermédiaire dépourvue d'électrodes, jusqu'aux électrodes de stimulation 34, regroupées autour d'une seconde région active Z₂, située à distance de la première région Z₁.

Avec cette configuration, il est possible non seulement de stimuler le ventricule gauche en plusieurs points de l'une des veines (du fait de la multiplication des électrodes 32 ou 34 définissant l'une ou l'autre des régions actives Z₁ et Z₂) mais en outre de prévoir deux régions relativement distantes de stimulation Z₁ et Z₂, respectivement la zone des électrodes 32 et celle des électrodes 34, situées dans des régions proximales de deux veines différentes dans lesquelles il aurait été difficile de stabiliser ou fixer des sondes conventionnelles de stimulation du ventricule gauche, du fait du grand diamètre de l'embouchure de ces veines.

Les électrodes 32 et/ou 34 sont constituées, comme on le précisera plus bas, de régions dénudées d'un conducteur périphérique isolé de l'extension tubulaire 26, et forment ensemble un réseau d'électrodes reliées ensemble, en série, permettant de multiplier les points de contact avec la paroi de la veine et d'assurer ainsi une diffusion multizones de l'énergie de stimulation en plusieurs points du réseau coronarien et donc du ventricule gauche.

La surface individuelle de chaque électrode est d'au plus 2 mm², ce qui permet d'en disposer un nombre important sans dépasser une surface totale cumulée de 10 mm². Du fait de la faible surface active cumulée, on bénéficie ainsi des avantages d'une sonde dite à "haute densité de courant", en termes à la fois d'efficacité physiologique de la stimulation et de moindre consommation d'énergie - ceci tout en maximisant les chances de contact physique, donc électrique, des électrodes 32, 34 avec les tissus excitables, du fait de la multiplication de ces électrodes.

La qualité du contact physique entre électrodes et tissus peut être sensiblement améliorée en préformant l'extension de manière à placer les électrodes au sommet d'ondulations, par exemple en forme de périodes de sinusoïde, correspondant aux zones respectives de stimulation.

Cette conformation est réalisée en usine, chaque ondulation étant une préforme donnée au conducteur spiralé (par exemple par 0traitement thermique sous déformation imposée) et/ou à la structure isolante à l'état libre, avec par exemple une longueur de la période de sinusoïde de l'ordre de 30 mm et une amplitude totale en direction radiale de l'ordre de 10 à 25 mm. Du fait de l'élasticité de la structure, les préformes des portions ondulées sont déformables sous contrainte radiale lors du passage dans les veines du réseau coronaire, et elles favorisent le contact des électrodes avec les tissus, et par là même la performance électrique de ces électrodes (notamment si ces électrodes sont situées au sommet des ondulations).

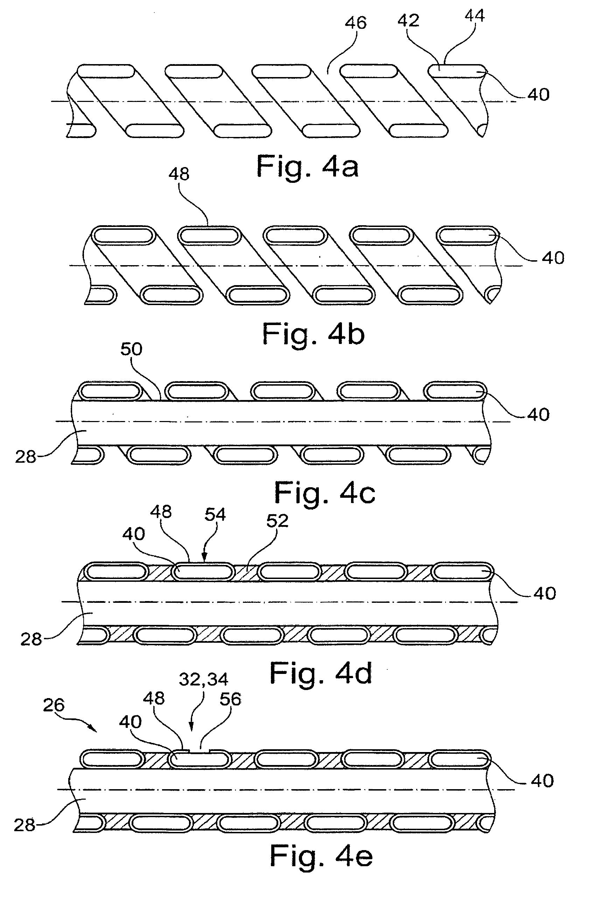

Les Figures 4a à 4e illustrent les étapes successives de réalisation de l'extension d'une sonde selon l'invention.

La première étape (Figure 4a) consiste à bobiner selon une configuration hélicoïdale un fil 40 en matériau conducteur. Le fil 40 est avantageusement un fil de section aplatie, qui lui confèrera une meilleure raideur axiale en traction et en compression, donc des propriétés mécaniques favorables à la *pushability,* c'est-à-dire l'aptitude de l'extension tubulaire 26 de la sonde à être avancée progressivement sur le fil-guide jusqu'au réseau veineux coronarien puis dans les tortuosités de celui-ci. Le fil 40 peut être notamment un fil de 0,08 x 0,16 mm de section formé d'un coeur 42 d'acier inoxydable, par exemple de MP35N, revêtu d'une gaine extérieure 44 en platine iridié. La fonction de ce revêtement est de créer une surface polaire apte à résister aux contraintes de corrosion sous circulation de courant. Ce fil est bobiné en spires non jointives, de manière à laisser subsister un intervalle 46, par exemple de l'ordre de 0,08 mm, entre spires successives.

A l'étape suivante (Figure 4b), le fil est soumis à un dépôt d'une couche isolante 48, constituée par exemple d'une fine couche de parylène de type C de 5 µm d'épaisseur.

L'étape suivante (Figure 4c) consiste à insérer un micro-tube 50 à l'intérieur de ce bobinage, par exemple un micro-tube de PTFE de 1,4 French (0,466 mm) de diamètre extérieur, laissant subsister en son centre une lumière interne 28 de l'ordre de 0,45 mm environ. Le matériau permet notamment un excellent glissement du fil-guide qui sera introduit dans la lumière 28, et assure en outre la protection de la lumière interne lors de l'étape suivante.

Cette étape suivante (Figure 4d) consiste à exécuter une opération de *backfilling,* c'est-à-dire de remplissage d'un matériau isolant 52, par exemple de silicone, dans les intervalles subsistant entre les spires du fil, tout en laissant apparente, en épargne, la face extérieure 54 du fil 40 recouvert de son isolant 48.

L'étape finale (Figure 4e) consiste à ménager des ouvertures 56 dans le gainage isolant 48, de manière à découvrir localement la partie conductrice du fil 40. On pourra notamment utiliser des techniques d'ablation par faisceau plasma ou laser, qui permettent des découpes très fines (jusqu'à 0,01 mm) dans l'isolant 48 en parylène, avec une très grande flexibilité dans le choix de la forme de la découpe 56 et le contrôle de son étendue. Il est ainsi possible de "programmer" une trajectoire de faisceau pour créer une électrode "longue" (jusqu'à 10 mm) de surface globale inférieure à 2 mm², augmentant encore la probabilité de contact avec le tissus sans perte de performance électrique. De même, dans le cas d'une version bipolaire que l'on décrira plus bas, il est possible de créer une électrode bipolaire longue mais de faible distance inter-électrode (typiquement au minimum le pas d'enroulement du conducteur de base)

Les régions localement dénudées 56 formeront les électrodes 32 et/ou 34 de stimulation constituant les parties actives de la sonde selon l'invention, situées sur l'extension tubulaire 26.

Les Figures 5 et 6 illustrent une variante de réalisation consistant à appliquer le revêtement isolant 48 de parylène sur l'ensemble de la structure, après le remplissage par le matériau isolant 52 (étape de la Figure 4d), en lieu et place de l'étape préliminaire de la Figure 4b. Les électrodes 32, 34, sont formées de la même façon que précédemment, par une découpe sélective du revêtement de parylène laissant apparaître les zones localement dénudées 56.

Une autre variante encore (non illustrée) consiste à bobiner non plus un, mais plusieurs fils, par exemple deux ou trois fils, de manière à conférer à l'extension 26 soit une propriété de redondance venant renforcer la fiabilité globale de la sonde, soit des capacités de multipolarité.

En effet, le procédé décrit ci-dessus préserve l'isolement électrique individuel des fils de l'extension par deux isolants redondants : parylène et silicone du *backfilling.* Il suffit alors préserver cet isolement en connectant individuellement ces deux fils aux deux conducteurs du corps de sonde (ce principe pouvant être étendu jusqu'à quatre pôles).

Un avantage notable de cette construction est la grande souplesse de réalisation des configurations des dipôles anode / cathode :
- position longitudinale des pôles le long de l'extension ;
- alternance des pôles au sein d'une même zone Z₁ ou Z₂ ;
- variation des surfaces anode/cathode sans aucune création de point de rigidité, contrairement aux constructions classiques.

Les Figures 7a à 7d illustrent divers modes de réalisation de l'invention, avec stimulation monopolaire ou bipolaire et/ou stimulation sur une ou sur deux zones actives :
- le mode de réalisation a) correspond à une stimulation monopolaire dans une seule zone ;
- le mode de réalisation b) correspond à une stimulation bipolaire dans une seule zone ;
- le mode de réalisation c) correspond à une stimulation monopolaire dans deux zones distantes (typiquement dans deux veines différentes) ;
- le mode de réalisation d) correspond à une stimulation bipolaire dans deux zones distantes.

Dans le cas d'une stimulation monopolaire, correspondant aux modes de réalisations a) et c), les impulsions sont délivrées respectivement : (i) entre d'une part le boîtier du générateur et d'autre part les électrodes 32, ou bien (ii) entre d'une part le boîtier du générateur et d'autre part les électrodes 32 et 34. Ces électrodes sont toutes reliées ensemble électriquement ainsi qu'à un connecteur 58, par exemple de type IS-1, situé à l'extrémité proximale de la sonde, destiné à être enfiché dans le boîtier.

Dans le cas d'une stimulation bipolaire, correspondant aux modes de réalisations b) et d), les impulsions sont délivrées respectivement :
- Figure 7b : entre d'une part une électrode additionnelle 60 disposée sur le corps de sonde et d'autre part les électrodes 32, ou
- Figure 7d : entre des électrodes 32 positives et une électrode 32' négative d'une première zone de stimulation, ainsi qu'entre des électrodes 34 positives et une électrode 34' négative d'une seconde zone de stimulation.

Le système de connexion est d'un type standard : le(s) fils de l'extension est(sont) connecté(s) en usine au(x) conducteur(s) du corps de sonde, lui(eux)-même(s) reliés aux bornes du connecteur 58.

Enfin, on notera que bien que dans les divers exemples on ait représenté la région active, ou chacune des deux régions actives, comme comprenant trois électrodes (32 et/ou 34), il est possible d'en prévoir un nombre inférieur ou supérieur d'électrodes dans chacune des zones actives correspondant aux régions Z₁ et/ou Z₂ où l'on souhaite opérer la stimulation. De même, bien que l'on ait décrit une stimulation sur une unique zone (Z₁ ou Z₂) d'une veine, ou sur une unique zone (Z₁, Z₂) de chacune de deux veines reliées par une anastomose, il est possible de prévoir un nombre supérieur de zones de stimulation sur chaque veine, par exemple si l'on souhaite stimuler l'une et/ou l'autre veine en plusieurs endroits.

On va maintenant exposer la technique d'implantation de la sonde selon l'invention.

Cette sonde est implantée par une technique conventionnelle de type OTW ou "filoguidage" au moyen d'un mandrin très fin formant fil-guide 30, pourvu à son extrémité distale d'une terminaison très souple pour ne pas être traumatique et permettre son introduction directe dans les vaisseaux du réseau coronarien sans risque de perforation.

Au préalable, le chirurgien dispose un cathéter principal lui permettant d'accéder au débouché du sinus coronaire, et un cathéter de sous-sélection permettant de choisir, sous amplificateur de brillance, le chemin du réseau veineux qui permettra d'atteindre la veine cible.

Le chirurgien introduit dans le cathéter de sous-sélection le fil-guide 30, qu'il pousse pour le faire progresser à nu dans le réseau veineux coronarien de façon à sélectionner telle ou telle veine collatérale, dans le cas présent la veine "aller" choisie (ici la veine antéro-latérale VA), puis - dans le cas d'une sonde permettant de stimuler deux veines différents - l'anastomose 26 et enfin la veine "retour" choisie (ici la veine postéro-latérale VPL) en remontant celle-ci. Le chirurgien enfile ensuite sur le fil-guide la sonde avec son corps de sonde 22 et son extension 26, qu'il fait alors glisser et progresser sur le fil-guide jusqu'à l'extrémité de ce dernier. Après retrait du fil-guide, le corps de sonde est soumis à un mouvement de vissage pour ancrer la spire de maintien 24 au débouché de la veine antéro-latérale VA.

La configuration est alors celle illustrée Figure 1, avec le groupe d'électrodes 32 (et 34 le cas échéant) disposées dans la région de stimulation Z₁ (et Z₂ aussi, le cas échéant) choisie.

Comme on peut le constater, ces étapes de cannulation de veine sont celles couramment pratiqués par les spécialistes de la technique de pose OTW, de sorte que l'implantation d'une sonde selon l'invention ne nécessite aucune nouvelle technique opératoire ni habileté particulières.

On remarquera que la solution de l'invention permet un placement optimal des électrodes de stimulation, grâce à la combinaison fil-guide 30/extension 26 du corps de sonde 22.

On notera en outre la simplicité et la robustesse de l'ensemble, malgré le très faible diamètre. En effet, sur l'extension, la ligne de conduction ne comporte aucune liaison critique telle que soudure ou collage assurant l'isolement électrique, cette ligne de conduction étant constituée d'un élément unique et robuste, à savoir le(s) fils de l'extension.

## Revendications

1. Une sonde de stimulation, destinée à être implantée dans le réseau veineux coronarien pour la stimulation d'une cavité du coeur, cette sonde (20) comprenant un corps de sonde (22) avec une gaine creuse traversée de part en part par une lumière centrale, l'extrémité distale du corps de sonde comprenant des moyens (24) de retenue à une paroi du réseau coronarien, sonde dans laquelle :
- le diamètre extérieur de l'extension est compris entre 1 et 3 French (0,33 et 1 mm) ;
- l'extrémité distale comporte à sa surface externe au moins un conducteur périphérique (40) électriquement isolé (48) à l'exception de zones ponctuellement dénudées (56) destinées à venir en contact avec la paroi d'une veine cible du réseau coronarien, de manière à constituer ainsi un réseau d'électrodes de stimulation (32, 34) reliées électriquement ensemble ;
- la sonde comporte une liaison électrique du conducteur périphérique (40) à des moyens (58), disposés côté proximal du corps de sonde, de couplage à un générateur de dispositif médical implantable actif tel que stimulateur cardiaque ou resynchroniseur,
- le corps de sonde (22) est prolongé à sa partie distale par une extension tubulaire creuse (26) solidaire du corps de sonde et portant une partie active de la sonde, cette extension étant traversée de part en part par une lumière centrale (28) communiquant avec la lumière centrale du corps de sonde de manière à y permettre l'introduction et le glissement d'un fil-guide (30) pour l'implantation de la sonde par une technique de filoguidage,
**caracterisée en ce que** :
- l'extension porte à sa surface externe ledit conducteur périphérique (40) électriquement isolé (48) à l'exception desdites zones ponctuellement dénudées (56).

2. La sonde de la revendication 1, dans laquelle le conducteur périphérique (40) est un fil bobiné sur un tube creux (50), ce fil étant électriquement isolé à l'exception des zones ponctuellement dénudées (56), formées côté extérieur du fil.

3. La sonde de la revendication 2, dans laquelle le fil bobiné est un fil plat.

4. La sonde de la revendication 2, dans laquelle le fil est bobiné en hélice à spires non jointives, l'intervalle (46) entre spires étant garni d'un matériau isolant de remplissage (52) sur l'épaisseur comprise entre la surface du tube creux et le niveau de la surface périphérique extérieure du fil bobiné.

5. La sonde de la revendication 2, dans laquelle le fil est un fil comprenant un coeur d'acier inoxydable (42) gainé de platine-iridium (44) et revêtu en surface d'une couche d'un matériau isolant (48).

6. La sonde de la revendication 1, dans laquelle la surface unitaire exposée des zones ponctuellement dénudées (56) du conducteur périphérique est d'au plus 2 mm².

7. La sonde de la revendication 1, dans laquelle la surface totale exposée des zones ponctuellement dénudées (56) du conducteur périphérique est d'au plus 10 mm².

8. La sonde de la revendication 1, dans laquelle les zones ponctuellement dénudées du conducteur périphérique sont regroupées sur au moins une région active (Z₁, Z₂) s'étendant sur une longueur comprise entre 1 et 5 cm.

9. La sonde de la revendication 1, dans laquelle le corps de sonde (22) porte côté distal au moins une électrode de stimulation bipolaire (60), non électriquement reliée au conducteur périphérique de l'extension.

10. La sonde de la revendication 1, dans laquelle les zones ponctuellement dénudées du conducteur périphérique sont regroupées sur deux régions actives (Z₁, Z₂) s'étendant chacune sur une longueur comprise entre 1 et 5 cm et séparées par une région intermédiaire non active de longueur comprise entre 5 et 15 cm.

11. La sonde de la revendication 1, dans laquelle l'extension comporte à sa surface externe deux conducteurs périphériques électriquement distincts, chacun d'entre eux étant relié à des ensembles respectifs distincts de zones ponctuellement dénudées (32, 34 ; 32', 34'), une même région active comprenant au moins une zone ponctuellement dénudée de chaque ensemble, de manière à permettre une stimulation bipolaire entre ces dernières.

12. La sonde de la revendication 1, dans laquelle l'extension comporte à son extrémité distale au moins un marqueur radio-opaque de repérage de la position distale de l'extension.

13. La sonde de la revendication 1, dans laquelle les moyens de retenue (24) à l'extrémité distale du corps de sonde comportent au moins un relief formé sur le corps de sonde, présentant localement un diamètre accru par rapport au diamètre propre du corps de sonde.

14. La sonde de la revendication 13, dans laquelle le relief est un relief hélicoïdal avec un filet s'enroulant autour du corps de sonde.

15. La sonde de la revendication 1, dans laquelle le diamètre du corps de sonde (22) est compris entre 2 et 5 French (0,66 et 2,66 mm).

16. La sonde de la revendication 1, dans laquelle le corps de sonde porte côté distal au moins un marqueur radio-opaque de repérage de la position proximale de l'extension.

## Patentansprüche

1. Stimulationssonde, die dazu bestimmt ist, in das Koronarvenennetz für die Stimulation eines Herzhohlraums implantiert zu werden, wobei diese Sonde (20) einen Sondenkörper (22) mit einer hohlen Hülle, die von einer Seite zur anderen von einem mittigen Langloch durchquert wird, umfasst, wobei das distale Ende des Sondenkörpers Mittel (24) zum Festhalten an einer Wand des Koronarnetzes umfasst, wobei in der Sonde:
- der Außendurchmesser der Erstreckung im Bereich von 1 bis 3 French (0,33 bis 1 mm) liegt;
- das distale Ende auf seiner äußeren Oberfläche wenigstens einen Umfangsleiter (40) aufweist, der mit Ausnahme von punktuell abisolierten Zonen (56), die dazu bestimmt sind, mit der Wand einer Zielvene des Koronarnetzes in Kontakt zu gelangen, elektrisch isoliert ist (48), derart, dass dadurch ein Netz von Stimulationselektroden (32, 34) gebildet wird, die alle elektrisch miteinander verbunden sind;
- die Sonde eine elektrische Verbindung des Umfangsleiters (40) mit Mitteln (58) aufweist, die auf der proximalen Seite des Sondenkörpers angeordnet sind, um mit einem Generator einer aktiven implantierbare medizinischen Vorrichtung wie etwa einem Herzstimulator oder einem Resynchronisator zu koppeln,
- der Sondenkörper (22) an seinem distalen Teil durch eine hohle rohrförmige Erstreckung (26) verlängert ist, die mit dem Sondenkörper fest verbunden ist und einen aktiven Teil der Sonde trägt, wobei diese Erstreckung von einer Seite zur anderen von einem mittiges Langloch (28) durchquert wird, das mit dem mittigen Langloch des Sondenkörpers kommuniziert, derart, dass hier die Einführung und das Gleiten eines Führungsdrahts (30) für die Implantation der Sonde durch eine Drahtlenkungstechnik ermöglicht wird,
**dadurch gekennzeichnet, dass**
- die Erstreckung auf ihrer äußeren Oberfläche den Umfangsleiter (40) trägt, der mit Ausnahme der punktuell abisolierten Zonen (56) elektrisch isoliert (48) ist.

2. Sonde nach Anspruch 1, wobei der Umfangsleiter (40) ein auf ein hohles Rohr (50) gewickelter Draht ist, wobei dieser Draht mit Ausnahme der Zonen, die punktuell abisoliert sind (56) und an der Außenseite des Drahts gebildet sind, elektrisch isoliert ist.

3. Sonde nach Anspruch 2, wobei der gewickelte Draht ein Flachdraht ist.

4. Sonde nach Anspruch 2, wobei der gewickelte Draht schraubenlinienförmig mit nicht nebeneinanderliegenden Windungen ist, wobei das Intervall (46) zwischen Windungen mit einem isolierenden Füllmaterial (52) mit einer Dicke zwischen der Oberfläche des hohlen Rohrs und der Höhe der äußeren Umfangsoberfläche des gewickelten Drahts versehen ist.

5. Sonde nach Anspruch 2, wobei der Draht ein Draht ist, der eine Seele aus Edelstahl (42) umfasst, die mit Platin-Iridium (44) umhüllt ist und an der Oberfläche mit einer Schicht (48) aus Isoliermaterial beschichtet ist.

6. Sonde nach Anspruch 1, wobei die einheitliche freiliegende Oberfläche der punktuell abisolierten Zonen (56) des Umfangsleiters höchstens 2 mm² beträgt.

7. Sonde nach Anspruch 1, wobei die gesamte freiliegende Oberfläche der punktuell abisolierten Zonen (56) des Umfangsleiters höchstens 10 mm² beträgt.

8. Sonde noch Anspruch 1, wobei die punktuell abisolierten Zonen des Umfangsleiters in wenigstens einer aktiven Zone (Z₁, Z₂), die sich über eine Länge im Bereich von 1 bis 5 cm erstreckt, angeordnet sind.

9. Sonde nach Anspruch 1, wobei der Sondenkörper (22) auf der distalen Seite wenigstens eine bipolare Stimulationselektrode (60) trägt, die mit dem Umfangsleiter der Erstreckung nicht elektrisch verbunden ist.

10. Sonde nach Anspruch 1, wobei die punktuell abisolierten Zonen des Umfangsleiters in zwei aktiven Bereichen (Z₁, Z₂), die sich jeweils über eine Länge im Bereich von 1 bis 5 cm erstrecken und durch einen nicht aktiven Bereich mit einer Länge im Bereich von 5 bis 15 cm getrennt sind, angeordnet sind.

11. Sonde nach Anspruch 1, wobei die Erstreckung auf ihrer äußeren Oberfläche zwei elektrisch getrennte Umfangsleiter umfasst, wobei jeder von ihnen mit entsprechenden verschiedenen Gesamtheiten punktuell abisolierter Zonen (32, 34; 32', 34') verbunden ist, wobei derselbe aktive Bereich wenigstens eine punktuell abisolierte Zone jeder Gesamtheit enthält, derart, dass zwischen diesen Letzteren eine bipolare Stimulation möglich ist.

12. Sonde noch Anspruch 1, wobei die Erstreckung an ihrem distalen Ende wenigstens eine für Funkwellen undurchlässige Markierung für die Kennzeichnung der distalen Position der Erstreckung aufweist.

13. Sondern nach Anspruch 1, wobei die Haltemittel (24) am distalen Ende des Sondenkörpers wenigstens ein auf dem Sondenkörper gebildetes Relief aufweisen, das einen in Bezug auf den eigentlichen Durchmesser des Sondenkörpers lokal erhöhten Durchmesser besitzt.

14. Sonde nach Anspruch 13, wobei das Relief ein schraubenlinienförmiges Relief mit einem Gewinde, das um den Sondenkörper verläuft, ist.

15. Sonde nach Anspruch 1, wobei der Durchmesser des Sondenkörpers (22) im Bereich von 2 bis 5 French (0,66 bis 2,66 mm) liegt.

16. Sonde nach Anspruch 1, wobei der Sondenkörper auf der distalen Seite wenigstens eine für Funkwellen undurchlässige Markierung für die Kennzeichnung der proximalen Position der Erstreckung aufweist.

## Claims

1. A stimulation lead, intended to be implanted in the coronary venous system for the stimulation of a cavity of the heart, said lead (20) comprising a lead body (22) with a hollow sheath pierced right through by a central lumen, the distal end of the lead body comprising means (24) for holding to a wall of the coronary system, a lead wherein:
- the external diameter of the extension is comprised between 1 and 3 French (0.33 and 1 mm);
- the distal end includes at its external surface at least one peripheral conductor (40) electrically insulated (48) except punctually naked areas (56) intended to come into contact with the wall of a target vein of the coronary system, so as to hence form a network of stimulation electrodes (32, 34) electrically connected to each other;
- the lead includes an electric connection of the peripheral conductor (40) to means (58), arranged on the proximal side of the lead body, for coupling to a generator of an active implantable medical device such as a cardiac stimulator or a resynchronizer,
- the lead body (22) is extended at its distal part by a hollow tubular extension (26) fastened to the lead body and carrying an active part of the lead, this extension being pierced right through by a central lumen (28) communicating with the central lumen of the lead body so as to allow the introduction and the sliding therein of a wire-guide (30), for the implantation of the lead by a wire-guiding technique,
**characterized in that**
- the extension carries at its external surface said peripheral conductor (40) electrically insulated (48) except said punctually naked areas (56).

2. The lead of claim 1, wherein the peripheral conductor (40) is a wire wound on a hollow tube (50), said wire being electrically insulated, except the punctually naked areas (56), formed on the external side of the wire.

3. The lead of claim 2, wherein the wound wire is a flat wire.

4. The lead of claim 2, wherein the wire is wound into a non-contiguous-turn coil, the interval (46) between turns being filled with a filling insulating material (52) over the thickness comprised between the surface of the hollow tube and the level of the external peripheral surface of the wound wire.

5. The lead of claim 2, wherein the wire is a wire comprising a stainless-steel core (42) sheathed with platinum-iridium (44) and surface-coated with a layer of insulating material (48).

6. The lead of claim 1, wherein the unitary exposed surface of the punctually naked areas (56) of the peripheral conductor is at most of 2 mm².

7. The lead of claim 1, wherein the total exposed surface of the punctually naked areas (56) of the peripheral conductor is at most of 10 mm².

8. The lead of claim 1, wherein the punctually naked areas of the peripheral conductor are grouped in at least one active region (Z₁, Z₂) extending over a length comprised between 1 and 5 cm.

9. The lead of claim 1, wherein the lead body (22) carries, on the distal side, at least one bipolar stimulation electrode (60), not electrically connected to the peripheral conductor of the extension.

10. The lead of claim 1, wherein the punctually naked areas of the peripheral conductor are grouped in two active regions (Z₁, Z₂) each extending over a length comprised between 1 and 5 cm and separated by a non-active intermediate region of length comprised between 5 and 15 cm.

11. The lead of claim 1, wherein the extension includes at its external surface two electrically distinct peripheral conductors, each of which being connected to respective distinct sets of punctually naked areas (32, 34 ; 32', 34'), a same active region comprising at least one punctually naked area of each set, so as to allow a bipolar stimulation between these latter.

12. The lead of claim 1, wherein the extension includes at its distal end at least one radio-opaque marker for marking the distal position of the extension.

13. The lead of claim 1, wherein the holding means (24) at the distal end of the lead body include at least one relief formed on the lead body, having locally an increased diameter with respect to the diameter of the lead body itself.

14. The lead of claim 13, wherein the relief is an helical relief with a thread winding up around the lead body.

15. The lead of claim 1, wherein the diameter of the lead body (22) is comprised between 2 and 5 French (0.66 and 2.66 mm).

16. The lead of claim 1, wherein the lead body carries, on the distal side, at least one radio-opaque marker for marking the distal position of the extension.
